# DEMANDE DE BREVET EUROPEEN

(11) **EP 1 413 625 A1**
(43) Date de publication de la demande: **28.04.2004**
(21) Numéro de dépôt: 03292373.2
(22) Date de dépôt: 26.09.2003
(51) Int. Cl.: C12N 5/00, A01N 1/02

(54) **Composition pour milieu biologique comprenant de l'érythorbate de sodium**

(30) Priorité: 16.10.2002 FR 0212879
(71) Demandeur: MACO PHARMA, 59420 Mouvaux (FR)
(72) Inventeur: Heron, Antoine, 59250 Halluin (FR)
(74) Mandataire: Derambure, Christian

(57) **Abrégé**

L'invention concerne une composition pour milieu biologique du type comprenant un anti-oxydant, dans laquelle l'anti-oxydant est de l'érythorbate de sodium.

L'invention a également pour objet un milieu biologique comprenant une telle composition.

## Description

L'invention concerne une composition pour milieu biologique comprenant un anti-oxydant, et un milieu biologique comprenant une telle composition.

Les milieux biologiques sont couramment utilisés en tant que milieux de culture cellulaire, ou en tant que milieux de lavage, de transport, de conservation ou de congélation d'organes ou de tissus.

Dans de tels milieux, il est souhaitable d'introduire un constituant exerçant une action anti-oxydante efficace. En effet, sortis de leur milieu physiologique, les cellules, tissus ou organes sont soumis à une tension d'oxygène anormalement élevée. Il est donc indispensable de lutter contre cette tension d'oxygène trop élevée et agressive à l'égard des éléments biologiques et cellulaires.

A cet effet, on connaît déjà des milieux biologiques comprenant des vitamines, telles que la vitamine C et la vitamine E, connues pour leur activité anti-oxydante. Toutefois, leur utilisation en tant qu'agent anti-oxydant est limitée par leur activité vitaminique, qui empêche d'utiliser des concentrations importantes de ces substances. En effet, les vitamines activent de multiples voies métaboliques, qui peuvent nuire aux fonctions essentielles des milieux concernés. Par exemple, l'emploi de vitamine C dans un milieu de conservation et de transport d'organes nuit à la fonction de conservation dudit milieu.

Pour pallier ces inconvénients, la demanderesse a effectué des essais intensifs pour trouver un agent anti-oxydant efficace ne présentant pas les inconvénients mentionnés ci-dessus.

La demanderesse a ainsi défini une composition et un milieu biologique comprenant, en tant qu'agent anti-oxydant, de l'érythorbate de sodium. Cette molécule présente notamment l'avantage de posséder la même activité antioxydante que l'ascorbate de sodium, sans posséder son activité vitaminique.

A cet effet, et selon un premier aspect, l'invention propose une composition pour milieu biologique du type comprenant un anti-oxydant, dans laquelle l'anti-oxydant est de l'érythorbate de sodium.

Selon un deuxième aspect, l'invention concerne un milieu biologique comprenant une telle composition.

D'autres objets et avantages de l'invention apparaîtront au cours de la description qui suit.

La composition selon l'invention est plus particulièrement destinée à des milieux biologiques, et notamment à des milieux de culture cellulaire ou des milieux de lavage, de transport, de conservation ou de congélation d'organes et de tissus.

Cette composition comprend un agent anti-oxydant. Il est en effet nécessaire, dans une telle application, de prévoir une substance possédant un pouvoir anti-oxydant important, et ce sans présenter les inconvénients inhérents par exemple à l'emploi de vitamines.

A cet effet, l'invention propose une composition dans laquelle l'agent anti-oxydant est de l'érythorbate de sodium. Cette molécule possède en effet les avantages mentionnés ci-dessus. En outre, elle possède un grade pharmaceutique et alimentaire, ce qui non seulement offre un gage de sécurité, mais permet en outre de l'utiliser sans limitation de concentration.

Par ailleurs, l'érythorbate de sodium est une molécule parfaitement soluble dans l'eau, ce qui rend sa manipulation aisée dans le cadre de l'application considérée.

Selon une réalisation, la quantité d'érythorbate de sodium utilisée dans la composition est inférieure ou égale à 0,1 % en poids, et est notamment inférieure à 5.10⁻² % en poids.

L'invention propose également un milieu biologique comprenant une telle composition.

En particulier, ce milieu peut être obtenu par dissolution de la composition. A cet effet, selon une première variante, il est possible de dissoudre, dans un volume donné de liquide, une composition préalablement formulée, c'est-à-dire de dissoudre simultanément l'ensemble des constituants d'une composition selon l'invention. Selon une deuxième variante, le milieu biologique peut être obtenu par dissolution séparée de certains constituants de la composition selon l'invention, de sorte à obtenir un milieu biologique dans lequel la concentration de chaque constituant correspond à la valeur requise.

Selon une réalisation, la concentration en érythorbate de sodium dans un tel milieu est inférieure ou égale à 500 mg/l.

Ce milieu biologique peut être un milieu de lavage, de transport, de conservation ou de congélation d'organes ou de tissus.

Dans de tels milieux, les propriétés de conservation doivent être identiques quel que soit l'organe à transplanter.

Plus précisément, il est nécessaire d'assurer une bonne viabilité et fonctionnalité de l'organe. Par exemple, dans le cas particulier de la transplantation rénale, une mauvaise conservation du greffon provoque des tubulopathies à l'origine de troubles postopératoires et un maintien prolongé du patient sous dialyse.

En outre, la conservation doit également prévenir l'oedème tissulaire, intracellulaire et interstitiel qui s'installe inévitablement pendant les longues périodes d'ischémie. Un oedème trop important (>40%) va provoquer une altération du cytosquelette et de la membrane plasmique, pouvant aboutir à des nécroses localisées.

Enfin, il est souhaitable de conserver les réserves énergétiques du greffon pour s'assurer de la bonne reprise de son activité métabolique au moment de la transplantation.

Cherchant à répondre à ces objectifs, différents types de milieux de lavage, de transport, de conservation ou de congélation, d'organes et de tissus, se sont développés. Même si pour certaines applications particulières, comme la conservation des cornées, ces milieux comportent du sérum de veau foetal, la stratégie actuelle est de se tourner vers des solutions salines équilibrées et des milieux définis sans sérum ou sans protéine pour éliminer tout risque potentiel de contamination et garantir une parfaite reproductibilité fonctionnelle.

Le problème qui se pose alors est que certes les risques de contamination et de non reproductibilité sont écartés, mais le milieu ne possède plus certaines propriétés bénéfiques du sérum, en particulier ses propriétés protectrices antioxydantes exercées notamment par la capture de radicaux libres.

La présence d'érythorbate de sodium dans de tels milieux est donc particulièrement bénéfique.

Le milieu biologique comprenant une composition selon l'invention peut également être un milieu de culture de cellules notamment animales ou de tissus.

Concernant ces milieux de culture, l'avènement des cultures *in vitro* de cellules directement transplantables chez l'homme est à l'origine du développement de différents types de milieux de culture.

Dans un premier temps, les milieux utilisés ont été des milieux indéfinis contenant du sérum. L'utilisation de sérum pose de nombreux problèmes : premièrement, la composition d'un milieu à base de sérum n'est que partiellement connue ; deuxièmement, le sérum est un vecteur potentiel d'agents pathogènes ; troisièmement, la standardisation du milieu est impossible, il y a une nette variabilité fonctionnelle entre les sérums et de lot à lot pour une même référence de sérum. Enfin, les sérums utilisés sont le plus souvent d'origine bovine, les risques de contamination notamment par les prions sont donc réels.

Plus récemment, face aux problèmes engendrés par les milieux indéfinis comportant du sérum, des milieux définis sans sérum ou « *serum-free* » ont fait leur apparition. Ces milieux sans sérum sont à base de substituts de sérum essentiels que sont l'albumine, la transferrine et l'insuline. Dans certaines applications, ces milieux peuvent être totalement synthétiques et ne comporter aucune protéine ; ils sont qualifiés de milieux « *protein-free* ».

La stratégie actuelle concernant les milieux de culture de cellules est donc de se tourner vers des milieux sans sérum, et même vers des substituts d'origine recombinante ou non naturelle afin d'éliminer tout risque potentiel de contamination et d'assurer une parfaire reproductibilité fonctionnelle.

Le problème qui se pose alors est que certes les risques de contamination et de non reproductibilité sont écartés, mais, de nouveau, le milieu ne possède plus certaines propriétés bénéfiques du sérum, en particulier ses propriétés protectrices antioxydantes exercées notamment par la capture de radicaux libres.

Là encore, l'utilisation d'érythorbate de sodium en tant qu'agent anti-oxydant est particulièrement bien adaptée.

Dans les deux applications mentionnées ci-dessus, le milieu biologique peut comprendre du polyéthylène glycol. En effet, ce polymère présente de nombreuses propriétés intéressantes à l'égard des cellules, des tissus et des organes. Et l'association d'érythorbate de sodium et de polyéthylène glycol est particulièrement intéressante dans les applications considérées, ce grâce à leurs actions protectrices complémentaires.

Cette action complémentaire vient du fait que l'érythorbate de sodium possède une action anti-oxydante sur le milieu biologique constituant l'environnement des cellules et tissus en culture, le polyéthylène glycol agissant principalement sur le maintien de l'intégrité structurelle et fonctionnelle desdits tissus et cellules, grâce à sa faculté de piéger les radicaux libres et de prévenir la péroxydation des lipides membranaires.

En outre, le polyéthylène glycol est capable d'exercer notamment des effets osmotiques, et peut ainsi par exemple limiter l'oedème tissulaire dans le cadre de la conservation de tissus et d'organes, ou encore substituer l'albumine dans le cadre de la culture cellulaire.

Par ailleurs, le polyéthylène glycol présente l'avantage de répondre pleinement aux exigences de qualité et de sécurité nécessaires pour un usage thérapeutique. En effet, ce polymère est couramment utilisé en tant que solvant, intermédiaire de synthèse ou excipient pour des préparations cosmétique et pharmaceutique, et possède de la sorte un grade pharmaceutique.

Selon une réalisation, la concentration en polyéthylène glycol est comprise entre 1 et 70 g/l, et notamment entre 10 et 30 g/l.

Le polyéthylène glycol utilisé est de poids moléculaire compris entre 50 et 100 000 Daltons, notamment 20 000 Daltons.

L'impact de l'érythorbate de sodium, indépendamment du PEG, a été évalué dans un milieu commercial de référence CellGro. Selon les indications du fournisseur de ce milieu de référence, il comporte des protéines humaines et des protéines humaines recombinantes, vraisemblablement de l'albumine, de la transferrine et de l'insuline recombinante. Des cellules hématopoïétiques primitives (CD34⁺) extraites de sang de cordon ombilical, ont été ensemencées à la concentration de 10000 cellules par ml dans ce milieu contenant des concentrations variables d'érythorbate de sodium (0 à 50 mg/L). Une combinaison de facteurs de croissance (cytokines) a été additionnée pour orienter la croissance de ces cellules : ce cocktail de cytokines est à base de SCF (50 ng/ml), TPO (50 ng/ml), FL (50 ng/ml), G-CSF (40 U/ml) et IL-6 (10 U/ml). Les cultures sont incubées 7 jours à 37°C dans une atmosphère saturée en humidité à 95% air/5% CO₂. Après 7 jours d'expansion, la numération et la viabilité cellulaire ont été déterminées. Les résultats sont exprimés dans le tableau 1.

**Tableau 1**

| **Milieu** | **Cellules à J0** | **Expansion à J6** | **Expansion à J7** | **Expansion à J8** |
|---|---|---|---|---|
| Milieu témoin | 10000 | 28,8x | 57,2x | 75,3x |
| | | *(94%)* | *(93%)* | *(81%)* |
| Milieu | 10000 | 30x | 56,8x | 83,5x |
| + Erythorbate 0,05 mg/L | | *(94%)* | *(91%)* | *(87%)* |
| Milieu | 10000 | 30,4x | 54,8x | 91,3x |
| + Erythorbate 0,5 mg/L | | *(95%)* | *(85%)* | *(86%)* |
| Milieu | 10000 | 36,8x | 65,6x | 94,6x |
| + Erythorbate 5 mg/L | | *(94%)* | *(91%)* | *(86%)* |
| Milieu | 10000 | 32,6x | 51,6x | 70,1x |
| + Erythorbate 50 mg/L | | *(92%)* | *(91%)* | *(85%)* |
| ** viabilité cellulaire correspondante* | | | | |

Ces résultats montrent premièrement que l'ajout d'érythorbate de sodium n'induit aucune forme de toxicité même à forte concentration. Les viabilités cellulaires restent excellentes dans chaque condition de culture (> 81%). Ils montrent également que l'on peut bonifier les qualités d'un milieu en terme d'expansion cellulaire (exemple : expansion à J8 en présence de 5 mg/L d'Erythorbate = 125% du témoin). Après 7 jours d'expansion, les cellules ont été ensemencées dans un milieu semi-solide (H4434, *StemCell Technologies*) afin de déterminer le taux d'expansion des progéniteurs clonogéniques. L'analyse des colonies obtenues est détaillée dans le tableau 2.

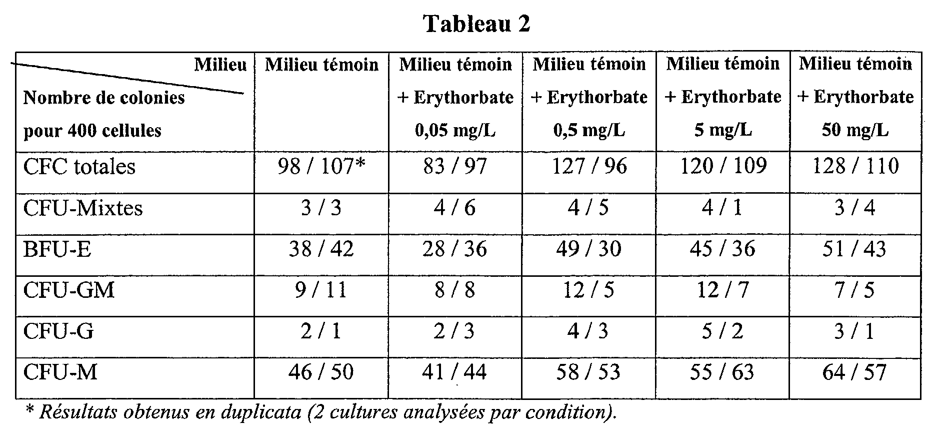

Les cultures clonogéniques ont alors démontré la qualité de l'érythorbate de sodium en terme d'amplification des progéniteurs clonogéniques (progéniteurs capables de donner une colonie de cellules *in vitro* dans un milieu semi-solide). L'indice de clonogénicité (nombre de colonies totales comptées pour 100 cellules ensemencées) n'est pas significativement très différent suivant les conditions d'expansion. La répartition entre les différents types de colonies est également similaire. La bonification du milieu de culture en terme d'expansion des cellules totales s'applique également à l'expansion des progéniteurs hématopoïétiques clonogéniques.

On décrit à présent à titre illustratif quatre exemples de milieux biologiques comprenant une composition selon l'invention. Les exemples 1 et 2 sont des milieux de culture cellulaire, et les exemples 3 et 4 sont des milieux pour le lavage, le transport, la conservation ou la congélation d'organes et de tissus.

### Exemple 1 :

Un milieu sans sérum d'expansion des cellules souches et progéniteurs hématopoïétiques a été défini selon la formule suivante :
- une base IMDM (*Iscove's Modified Dulbecco's Medium*) composée de : CaCl₂ anhydre (165 mg/L), KCI (330 mg/L), KNO₃ (0,076 mg/L), MgSO₄ anhydre (97,67 mg/L), NaCl (4505 mg/L), NaHCO₃ (3024 mg/L), NaH₂PO₄.H₂O (125 mg/L), Na₂SeO₃.5H₂O (0,01 mg/L), Glucose (4500 mg/L), HEPES (5958 mg/L), rouge de phenol.Na (15 mg/L), pyruvate de sodium (110 mg/L), L-Alanine (25 mg/L), L-Arginine.HCl (84 mg/L), L-Asparagine.H₂O (28,40 mg/L), L-Aspartic acid (30 mg/L), L-Cystine .2HCl (91,24 mg/L), L-Glutamic acid (75 mg/L), L-Glutamine (584 mg/L), Glycine (30 mg/L), L-Histidine.HCl.H2O (42 mg/L), L-Isoleucine (105 mg/L), L-Leucine (105 mg/L), L-Lysine.HCl (146 mg/L), L-Methionine (30 mg/L), L-Phenylalanine (66 mg/L), L-Proline (40 mg/L), L-Serine (42 mg/L), L-Threonine (95 mg/L), L-Tryptophan (16 mg/L), L-Tyrosine.2Na.2H₂O (104,20 mg/L), L-Valine (94 mg/L), D-Biotin (0,013 mg/L), D-Ca Pantothenate (4 mg/L), Choline Chloride (4 mg/L), Folic Acid (4 mg/L), i-Inositol (7,2 mg/L), Nicotinamide (4 mg/L), Pyridoxine.HCl (4 mg/L), Riboflavin (0,4 mg/L), Thiamine.HCl (4 mg/L), Vitamin B₁₂ (0,013 mg/L),
- de l'insuline humaine recombinante (1-100 mg/L) ou un substitut d'insuline tel que le chlorure de zinc (0,1-10 mg/L),
- du gluconate de fer (II) (50-1000 mg/L),
- de l'albumine humaine injectable (0,1 à 2%),
- de l'Erythorbate de sodium (1-50 mg/L),
- des compléments spécifiques pour l'expansion de ces cellules tels que : Glutathion réduit (0,01-0,1 mg/L) et nucléosides (0,1-10 mg/L).

L'addition de PEG, notamment de PEG 20000 Daltons (10-30 g/L) dans ce premier exemple de formule de milieu selon l'invention, vient renforcer les propriétés protectrices de ce milieu à l'égard des cellules et tissus cultivés. Dans certaines applications de culture de cellules humaines à visée thérapeutique, l'incorporation de PEG 20000 Daltons vient également substituer tout ou partie des propriétés fonctionnelles de l'albumine permettant ainsi de limiter la concentration voire d'éliminer totalement l'albumine de cette formule selon l'invention.

### Exemple 2 :

Un milieu sans protéine pour la culture industrielle d'hybridomes producteurs d'anticorps monoclonaux a été défini selon la formule suivante :
- une base RPMI 1640 composée de : Ca(NO₃)₂.4H₂O (100 mg/L), KCI (400 mg/L), MgSO₄.7H₂O (100 mg/L), NaCl (5000 mg/L), NaHCO₃ (2000 mg/L), Na₂HPO₄.7H₂O (1512 mg/L), Glucose (2000 mg/L), Glutathione (reduced) (1 mg/L), HEPES (5957,4 mg/L), rouge de phenol.Na (5 mg/L), L-Arginine (200 mg/L), L-Asparagine.H₂O (50 mg/L), L-Aspartic acid (20 mg/L), L-Cystine (50 mg/L), L-Glutamic acid (20 mg/L), L-Glutamine (300 mg/L), Glycine (10 mg/L), L-Histidine (15 mg/L), Hydroxy-L Proline (20 mg/L), L-Isoleucine (50 mg/L), L-Leucine (50 mg/L), L-Lysine.HCl (40 mg/L), L-Methionine (15 mg/L), L-Phenylalanine (15 mg/L), L-Proline (20 mg/L), L-Serine (30 mg/L), L-Threonine (20 mg/L), L-Tryptophan (5 mg/L), L-Tyrosine (20 mg/L), L-Valine (20 mg/L), p-Aminobenzoic Acid (1 mg/L), D-Biotin (0,2 mg/L), D-Ca Pantothenate (0,25 mg/L), Choline Chloride (3 mg/L), Folic Acid (1 mg/L), i-Inositol (35 mg/L), Nicotinamide (1 mg/L), Pyridoxine.HCl (1 mg/L), Riboflavin (0,2 mg/L), Thiamine.HCl (1 mg/L), Vitamin B₁₂ (0,005 mg/L),
- un substitut d'insuline tel que le chlorure de zinc (0,1-10 mg/L),
- du gluconate de fer (II) (50-1000 mg/L),
- du PEG 20000 Daltons (10-30 g/L),
- de l'érythorbate de sodium (1-50 mg/L),
- des compléments spécifiques pour l'expansion des hybridomes tels que : nucléosides (0,1-10 mg/L), pyruvate de sodium (1-100 mg/L), Putrescine.2HCl (0,05-5 mg/L), Hypoxanthine (0,1-10 mg/L).

Pour la culture d'hybridomes producteurs d'anticorps monoclonaux, les bases IMDM et DMEM/HAM-F12 (1/1) sont également recommandées. Les compléments du milieu selon l'invention sont alors définis en fonction de la base utilisée : ainsi, la base DMEM/HAM-F12 intègre directement des molécules telles que la Putrescine, l'Hypoxanthine, la Thymidine ou bien encore du pyruvate de sodium.

En matières de compléments spécifiques, les milieux biologiques selon l'invention, notamment destinés aux applications de culture cellulaire, n'excluent pas l'utilisation de molécules d'origine végétale. Le cas échéant, des lipides insolubles peuvent être aussi additionnés au milieu selon l'invention, sous une forme libre ou complexée, notamment avec des cyclodextrines. Le PEG décrit dans le cadre de l'invention peut également constituer un vecteur desdits lipides ou de toute autre molécule, après avoir été couplé suivant un procédé de « pegylation ».

### Exemple 3 :

L'intégration d'érythorbate de sodium dans un milieu biologique de type Krebs pour le lavage, le transport, la conservation ou la congélation, d'organes et de tissus, a été testé sur la base de la formulation suivante :
- Chlorure de sodium : 115,5 mM
- Chlorure de potassium : 4,63 mM
- Chlorure de calcium : 2,47 mM
- Chlorure de magnésium : 1,16 mM
- Bicarbonate de sodium : 21,9 mM
- EDTA, Na : 0,01 mM
- Glucose : 11 mM
- Sodium dihydrogénophosphate: 1,16 mM
- Erythorbate de sodium : de 0 à 500 mg/l

De façon conventionnelle, ces solutions de type Krebs comportent de la vitamine C. La substitution de la vitamine C par de l'érythorbate de sodium prend toute sa signification dans ce type de solutions au regard des propriétés de l'érythorbate précédemment décrites.

### Exemple 4 :

L'intégration d'érythorbate de sodium en association avec du PEG dans un milieu biologique pour le lavage, le transport, la conservation ou la congélation, d'organes et de tissus, a été testée sur la base d'une formulation décrite dans le brevet FR 2723818 :
- Chlorure de sodium: de 50 à 170 mM
- Chlorure de potassium : de 3 à 140 mM
- Chlorure de calcium : de 0 à 6 mM
- Chlorure de magnésium : de 0 à 50 mM
- Bicarbonate de sodium : de 0 à 70 mM
- EGTA : de 0 à 5 mM
- Glucose : de 0 à 200 mM
- Erythorbate de sodium : de 0 à 500 mg/l
- PEG 20000 Daltons : de 5 à 70 g/l

Dans ce type de solution de conservation d'organe, la présence d'érythorbate de sodium vient renforcer les propriétés protectrices notamment antioxydantes du milieu biologique, à l'égard des tissus et organes d'intérêts et conservés dans ce milieu.

Les milieux biologiques selon l'invention et plus particulièrement les milieux de lavage, de transport, de conservation ou de congélation, d'organes et de tissus trouvent notamment des applications dans :
- le transport et la conservation à 4°C d'organes en vue de transplantation, notamment dans la cadre de la transplantation du rein, du coeur, du foie ou du poumon,
- le transport et la conservation à température ambiante et plus (exemples : incubation à 31°C ou 37°C) de tissus en vue de transplantation, notamment dans le cadre de la greffe des cornées,
- la cryopréservation de tissus et organes, notamment dans le cadre de la conservation des gros tronc artériels, des valves ou de la peau,
- le lavage peropératoire de la cavité thoracique ou abdominale.

Les milieux biologiques selon l'invention et plus particulièrement les milieux de culture selon l'invention trouvent notamment des applications dans :
- la culture de cellules souches somatiques, notamment les cellules souches et progéniteurs hématopoïétiques issus de la moelle osseuse, du sang périphérique ou du sang de cordon ombilical,
- la culture et l'activation de cellules immunitaires, notamment les lymphocytes tels que : PBL (*peripheral blood lymphocytes*) ; LAK (*lymphokine activated killer cells*) ; TIL (*tumor infiltrating lymphocytes*),
- la culture des monocytes et macrophages,
- la production de cellules présentatrices d'antigènes, notamment les cellules dendritiques,
- la culture des hépatocytes, notamment pour la transplantation ou la préparation d'un foie bioartificiel,
- la culture des îlots de Langerhans, notamment dans le cadre du traitement des diabètes,
- l'expansion et la différentiation des cellules souches mésenchymateuses, notamment dans le cadre de la réparation des déficits osseux,
- la culture de cellules musculaires, notamment dans le cadre du traitement des déficiences cardiaques,
- la culture des cellules neuronales, somatiques, foetales ou embryonnaires, notamment dans le cadre du traitement des maladies neurodégénératives,
- la culture des cornées ou autres tissus,
- la culture de cellules souches embryonnaires,
- la décongélation, le lavage et la congélation des cellules et tissus à visée thérapeutique.

Les milieux biologiques selon l'invention et plus particulièrement les milieux de culture selon l'invention s'ouvrent également à un vaste champ d'applications dans le domaine industriel biotechnologique notamment pour :
- la culture d'hybridomes producteurs d'anticorps monoclonaux, notamment lorsque ces anticorps sont directement injectables chez l'homme,
- la préparation de molécules produites par culture de cellules, notamment les nombreuses lignées transfectées ou non de myélomes et CHO (*Chinese Hamster Ovary cells*), à des fins thérapeutique, vaccinale, diagnostique ou de recherche.
- la préparation d'entités biologiques par culture de cellules, notamment la production de vecteurs viraux au service des thérapies cellulaires.

## Revendications

1. Composition pour milieu biologique du type comprenant un anti-oxydant, caractérisée en que l'anti-oxydant est de l'érythorbate de sodium.

2. Composition selon la revendication 1, **caractérisée en ce que** la quantité d'érythorbate de sodium est inférieure ou égale à 0,1 % en poids, notamment inférieure à 5.10⁻² % en poids.

3. Milieu biologique, **caractérisé en ce qu'**il comprend la composition selon la revendication 1 ou 2.

4. Milieu biologique selon la revendication 3, **caractérisé en ce qu'**il est obtenu par dissolution de la composition.

5. Milieu biologique selon la revendication 3 ou 4, **caractérisé en ce que** la concentration en érythorbate de sodium est inférieure ou égale à 500 mg/l.

6. Milieu biologique selon l'une des revendications 3 à 5, **caractérisé en ce que** ledit milieu est un milieu de lavage, de transport, de conservation ou de congélation d'organes ou de tissus.

7. Milieu biologique selon l'une des revendications 3 à 5, **caractérisé en ce que** ledit milieu est un milieu de culture de cellules notamment animales ou de tissus.

8. Milieu biologique selon la revendication 6 ou 7, **caractérisé en ce qu'**il comprend en outre du polyéthylène glycol.

9. Milieu biologique selon la revendication 8, **caractérisé en ce que** la concentration en polyéthylène glycol est comprise entre 1 et 70 g/l, notamment entre 10 et 30 g/l.

10. Milieu biologique selon l'une des revendications 8 ou 9, **caractérisé en ce que** le polyéthylène glycol est de poids moléculaire compris entre 50 et 100 000 Daltons, notamment 20 000 Daltons.
